# EUROPEAN PATENT SPECIFICATION

(11) **EP 3 095 466 B1**
(45) Date of publication and mention of the grant of the patent: **16.12.2020**
(21) Application number: 16020185.1
(22) Date of filing: 21.05.2016
(51) Int. Cl.: A61K 9/00, A61K 47/12, A61K 47/26, A61K 45/06, A61K 31/167, A61K 31/137

(54) **PHARMACEUTICAL FORMULATIONS WITH IMPROVED SOLUBILITY AND STABILITY**
LÖSLICHKEITS- UND STABILITÄTSVERBESSERTE PHARMAZEUTISCHE ZUSAMMENSETZUNGEN
FORMULATIONS PHARMACEUTIQUES AVEC SOLUBILITÉ ET STABILITÉ AMÉLIORÉE

(30) Priority: 21.05.2015 TR 201506118
(43) Date of publication of application: 23.11.2016
(73) Proprietor: Abdi Ibrahim Ilac Sanayi ve Ticaret A.S., 34500 Esenyurt/Istanbul (TR)
(72) Inventor: FARSI, Ferhad, 34500 Esenyurt /Istanbul (TR); DUDE, Udaya Kumar, 34500 Esenyurt /Istanbul (TR); KOC, Erhan, 34500 Esenyurt /Istanbul (TR); ISIK, Enis, 34500 Esenyurt /Istanbul (TR); AKDAG, Kadriye, 34500 Esenyurt /Istanbul (TR)

(56) References cited:
- US-A1- 2004 162 273
- US-A1- 2007 141 144
- Novartis Consumer Health Canada: "NeoCitran Cold & Flu Night, Patient information leaflet", , 1 January 2013 (2013-01-01), pages 1-1, XP55308345, Retrieved from the Internet: URL:http://www.neocitran.ca/productinfo/rs cf_label_en.pdf [retrieved on 2016-10-06]

## Description

### Technical Field of Invention

The present invention relates to a pharmaceutical formulations to be used in the symptomatic treatment of respiratory tract infections such as cold and flu.

### Background of the Invention

The mucosal congestion of upper respiratory tracts caused by respiratory tract infections such as cold and flu may lead to many nasal and ocular symptoms. These may include symptoms such as sinusitis, nasal and sinus congestions or hypersecretion, headache, sneeze, etc.

Nowadays these symptoms are treated with pharmaceutical compositions comprising decongestants or combinations thereof with analgesic agents.

Phenylephrine is an alpha-adrenergic receptor agonist that is commonly used in diseases such as cough and cold due to its decongestant action.

Phenylephrine and its synthesis were first described in the patent document numbered US1932347 (1933; Frederick Steams). Its chemical name is (S)-1-(3-Hydroxyphenyl)-2-methylaminoethanol and its chemical structure is shown in formula I. It is freely soluble in water and alcohol; it is practically insoluble in chloroform and ether.

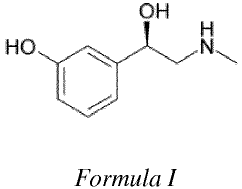

Phenylephrine relieves nasal congestion due to allergy or cold as a result of constriction of vessels in the nasal mucosa following oral administration. After oral administration, the decongestion, starting in 15-20 minutes, acts up to 4 hours.

Also, pheniramine, like phenylephrine, is a commonly used as decongestant and due to its property, it is used for symptomatic treatment of cough and cold.

Pheniramine was first described in the patent document numbered US2567245 (1951; Schering Corp). This document discloses synthesis of pheniramine and use of pheniramine in allergic reactions. Its chemical name is N,N-Dimethyl-3-phenyl-3-(2-pyridyl)propylamine hydrogen and its chemical structure is as shown in formula II.

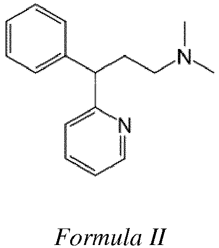

After oral administration, maximum plasma concentrations are reached within 1 or 2.5 hours. Bioavailability of pheniramine is 100% when taken orally.

One of the analgesic agents used in combination with decongestants such as phenylephrine and/or pheniramine is paracetamol. Paracetamol, also known as acetaminophen, is chemically named as *N*-(4-Hydroxyphenyl)ethanamide and has the structure as shown in formula III. It was first described in the patent document numbered US2998450 (1961; Warner-Lambert Pharmaceutical).

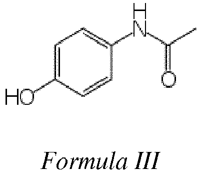

Although the exact mechanism of action of paracetamol is not known, it mainly acts on the central nervous system (CNS) and inhibits cyclooxygenase enzyme related to the synthesis of prostaglandin (PG). It may also inhibit the synthesis or activities of chemical intermediates, which increase the sensitivity of pain receptors against mechanical and chemical stimulants. When it is given orally, it is rapidly and almost fully absorbed through the gastrointestinal tract. It reaches its maximum plasma concentrations in 30-60 minutes after being taken. In addition to the abovementioned decongestants and analgesic agents, vitamins, especially vitamin C, are commonly used in the symptomatic treatment of respiratory tract infections such as cold and flu.

Vitamin C, also known as ascorbic acid, is a water-soluble vitamin found in fruits and vegetables such as *citrus* types (such as lemon, orange) and green pepper. It is essential for keeping tissues in the body in a healthy state, for particularly plerosis and collagen production.

Its chemical name is enolic form of 3-oxo-L-gulofuranolactone and its structure is as shown in formula IV.

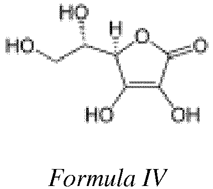

In the prior art, many patent documents describe the formulations, which comprise several combinations of the said agents to be used in the symptomatic treatment of respiratory tract infections such as cold and flu, were described.

WO2007125501 discloses the liquid dosage forms with a pH between 6.5 and 7.5 comprising phenylephrine and paracetamol were described.

WO2014120021 discloses a formulation comprising specific amounts of phenylephrine and paracetamol.

RU2166937 discloses a formulation comprising phenylephrine, paracetamol and ascorbic acid and sucrose or glucose, polyvinylpyrrolidone, sweetening and flavoring agents was described.

RU2237475 discloses combination of a compound selected from the group of metamizole, paracetamol and ibuprofen; an antihistaminic compound selected from the group of promethazine, pheniramine, terfenadine, astemizole, acrivastine, loratadine, fexofenadine and phenylephrine; and ascorbic acid for eliminating cold and flu symptoms.

In addition to these documents, the pharmaceutical dosage forms comprising different combinations of the said active ingredients are available in the market.

### For example;

- The product named Corsal® capsule and syrup comprises paracetamol, phenylephrine hydrochloride, chlorpheniramine maleate;
- The product named Deflu® tablet and syrup comprises paracetamol, phenylephrine hydrochloride, chlorpheniramine maleate;
- The product named Theraflu® Forte tablet comprises paracetamol, phenylephrine hydrochloride, chlorpheniramine maleate.

As clearly seen from the examples given, none of these dosage forms comprise a combination of active ingredients with ascorbic acid.

The product named NeoCitran Grippe® of the company Novartis Consumer Health Schweiz AG comprises 10 mg phenylephrine, 20 mg pheniramine, 500 mg paracetamol and 50 mg ascorbic acid. The said drug is supplied in sachets comprising water-soluble granules. The sachet dosage form often used in the pharmaceutical technology is an alternative option especially for children, the elderly, or patients who have difficulties with oral administration of solid dosage forms.

However, some difficulties are experienced when formulating sachet dosage forms. The favorable therapeutic efficacy in such soluble dosage forms can be obtained only in cases where the active ingredient and final product have high solubility. In other words, while the dosage forms such as sachets can easily be applied to active ingredients with high solubility, difficulties are experienced when applying them to active ingredients with low solubility.

Also, the parameters such as water-solubility, dissolution rate and dissolution time of the sachet form prepared lead to significant changes in the efficacy of the finished dosage form. Namely, the insufficiently solubility of the sachet form cause decreasing therapeutically dose amount and opaque solution which is undesirable to be drunk by the patient. Therefore therapeutically patient compliance is reduced and so therapeutic efficacy is decreased.

For example, the low water solubility of the product named NeoCitran Grippe® results in an opaque appearance.

Another problem faced with sachet dosage forms is that obtained dosage forms do not have a drinkable taste since some active ingredients have a bitter taste. In order to suppress the bitter taste, sweetening (flavor modifier/masker/suppressor) agents, a sugar derivative, are usually used in high quantities in the formulations. For example; the product named NeoCitran Grippe® comprises sugar equal to 340 kJ (82 kcal) per 20 g sachet. This sugar quantity corresponds to about 4 sugar cubes, and it prevents especially in individuals, need to balance usage of sugar (for example; patients with diabetes; low-calorie dieting), use of like the dosage form.

On the other hand, formulations having of high quantities of sweetening agents are not preferred, because it causes difficulty in packaging due to increasing the weight of the finished dosage form and increases the cost of the final product

Due to the abovementioned problems, new dosage form, which may be an alternative to the dosage forms found in the art, have improved solubility, have high therapeutic efficacy and popular in a large profile of patients, is needed to be developed.

The aim of the inventors is to develop stable, water-soluble formulations which comprise phenylephrine, pheniramine, paracetamol and ascorbic acid with improved solubility properties; dissolve in the desired time and in the desired quantity and create a clear solution; are suitable for being used by all patient groups; and are easy to produce and low-cost.

### Description of the Invention

The object of the invention is to develop new stable formulations, which comprise phenylephrine, pheniramine, paracetamol, and ascorbic acid with improved solubility properties; and dissolve in the appropriate time and in the appropriate quantity and create a clear solution.

The inventors have overcome the problems such as dissolution quantity, dissolution rate and dissolution time experienced in sachet formulations found in the art, as a result of the studies they developed a new water-soluble dosage form with optimum therapeutic efficacy and patient compliance.

As a result of the studies, the inventors have found out that the product in sachets comprising phenylephrine, pheniramine, paracetamol and ascorbic acid as active ingredients did not fully dissolve in water and the dissolution of the said product in water had an opaque appearance. The detailed studies performed by the inventors have indicated that the opaque appearance resulted from the failure of the active ingredient paracetamol to dissolve in the desired time and in the desired quantity. As a result of the long-term studies, the inventors surprisingly have found out that the active ingredient paracetamol having a D90 value of 200 to 400 µm, preferably 200 to 300 µm, more preferably 200 to 250 µm had optimum properties for the water-soluble formulation and overcame the abovementioned problems.

While paracetamol having a D90 value of more than 400 µm does not cause an efficient dissolution of the final pro duct, paracetamol with a D90 value of less than 200 µm dissolves well in water, but it tends to agglomerate during production and cannot have the desired flow properties. Therefore, it cause to not provide content uniformity during production and finished product.

The term "D90 value" used throughout the text refers to the particle size that 90% of particles have in volume. The D90 value of the active ingredient paracetamol can be measured using the sieving method or, in order to obtain a more precise result, the device measuring the particle distribution with laser diffraction (for example; Malvern Mastersizer).

The formulations object of the present invention comprise paracetamol of 10 to 1000 mg, preferably 250 to 625 mg, per unit dosage form. Based on the preferred arrangement of the invention, the formulations comprise paracetamol of 500 mg per unit dosage form.

The formulations object of the present invention comprise phenylephrine of 5 to 50 mg or a pharmaceutically acceptable salt thereof per unit dosage form.

According to preferred object of the invention, the formulations comprise 10 mg phenylephrine or a salt thereof per unit dosage form. The said phenylephrine salts may be selected from hydrochloride, hydrobromide, bitartrate and tannate salts. In the formulation object of the invention, phenylephrine hydrochloride was used preferably.

Another object of the invention, especially phenylephrine hydrochloride equivalent to 10 mg phenylephrine was used preferably.

In yet another object of the invention, the formulation comprise 10 to 100 mg pheniramine or a pharmaceutically acceptable salt thereof in the per unit dosage form, besides to paracetamol and phenylephrine as active ingredients.

In a further specific object of the invention, the formulations comprise 20 mg pheniramine or a salt thereof in the per unit dosage form.

The pheniramine salt used in the formulations object to the invention may 1 be pheniramine maleate.

According to another preferred object of the invention, the pheniramine salt used in the formulations is pheniramine maleate.

In the object of the invention, especially pheniramine maleate equivalent to 20 mg pheniramine was used preferably.

In another object of the invention, the formulations comprise 10 to 200 mg of ascorbic acid, preferably 20 to 100 mg per unit dosage form, besides to paracetamol, phenylephrine and pheniramine as active ingredients.

In a preferred object of the invention, the formulation comprises ascorbic acid 50 mg.

In other words, the formulations based on the invention comprise:
- Paracetamol
- Phenylephrine or a pharmaceutically acceptable salt thereof
- Pheniramine or a pharmaceutically acceptable salt thereof
- Ascorbic acid as active ingredients.

On the other hand, the formulations based on the invention comprise:
- Paracetamol
- Phenylephrine hydrochloride
- Pheniramine maleate
- Ascorbic acid
as active ingredients.

The object of the present invention formulations further comprise at least one pharmaceutically acceptable excipient which is mannitol.

The additional excipients can be selected from, but not limited to, binder, diluent, lubricant, disintegrant, stabilizer, flavoring, sweetener and/or coloring agents.

The binder can be selected from, but is not limited to, cellulose, ethyl cellulose, hydroxymethyl cellulose, hydroxypropyl methylcellulose, lactose, maltose, mannitol, polyvinylpyrrolidone.

The diluent can be selected from, but is not limited to, starch, lactose, maltose, mannitol, dextrose, fructose, lactilol, maltilol, maltodextrin, sorbitol, microcrystalline cellulose, calcium carbonate, dibasic calcium phosphate, tribasic calcium phosphate, calcium sulfate and magnesium carbonate.

An aspect of the invention, the preferred diluent agent is mannitol. The formulation of the present invention comprises mannitol of 1000 to 20000 mg, preferably 3000 to 8000 mg. When the stability of the reference product is controlled, it is seen that the assay of ascorbic acid was out of specification at 40 °C in the 1^{st} month and at 30 °C in the third month.

Due to its non-hygroscopic structure, using mannitol as a diluent provides a positive contribution to the stability of moisture sensitive active agents such as vitamin C.

The lubricant can be selected among stearic acid, magnesium stearate, polyethylene glycol, and sodium lauryl sulfate.

The disintegrant agent to can be selected from, but is not limited to, a group consisting of carboxymethyl cellulose, hydroxypropyl cellulose, hydroxypropyl methylcellulose, polyvinylpyrrolidone, crospovidone, microcrystalline cellulose, sodium starch glycolate, alginic acid, sodium alginate, or combination thereof. Also, the formulation of the object of the invention can include effervescent acid and effervescent base as disintegrant agent. The effervescent acid can be selected from, but is not limited to, a group consisting of citric acid, tartaric acid, adipic acid, succinic acid, malic acid, fumaric acid; and/or pharmaceutically acceptable salts, hydrates and anhydrates thereof; or mixtures thereof. The effervescent base can be selected from, but is not limited to, a group consisting of sodium, potassium and calcium carbonates, bicarbonates; or hydrates and anhydrates thereof; or mixtures thereof.

The stabilizer agent can be selected among, but is not limited to citric acid, malic acid, sodium citrate, cyclodextrin and tetrasodium edetate.

In specific object of the invention, preferably citric acid anhydride and sodium citrate anhydride is used in the formulation.

In a specific object of the invention, the formulation comprises 100 to 15000 mg citric acid, preferably 200 to 1000 mg.

In a further specific object of the present invention, the formulation comprises 200 to 4000 mg sodium citrate anhydride, preferably 500 to 1500 mg.

The flavoring agent can be selected from, but is not limited to, a group consisting of banana, strawberry, lemon, orange, peach, vanilla or a similar natural flavoring agent or mixtures thereof.

The sweetener agent can be selected from, but is not limited to, a group consisting of aspartame, dextrose, fructose, sucralose, sodium cyclamate, mannitol, maltose, sorbitol, saccharine or combination thereof.

Based on the invention, the formulations comprise:
A. As active ingredients;
   - Paracetamol
   - Phenylephrine or a pharmaceutically acceptable salt thereof,
   - Pheniramine or a pharmaceutically acceptable salt thereof,
   - Ascorbic acid;
B. As excipients;
   - citric acid anhydride,
   - sodium citrate anhydride and
   - mannitol.

Specifically based on the invention, the formulations comprise:
A. As active ingredients;
   - Paracetamol
   - Phenylephrine hydrochloride
   - Pheniramine maleate and
   - Ascorbic acid;
B. As excipients;
   - citric acid anhydride,
   - sodium citrate anhydride and
   - mannitol.

More specifically based on the invention, the formulations comprise:
- 5 to 50 mg Phenylephrine hydrochloride
- 10 to 100 mg Pheniramine maleate
- Paracetamol 10 to 1000 mg
- 10 to 200 mg Ascorbic acid
- 100 to 1500 mg Citric acid anhydride
- 200 to 4000 mg Sodium citrate anhydride
- 1000 to 20000 mg Mannitol
- at least one pharmaceutically acceptable excipient which is mannitol 1 to 5000 mg.

In another object of the invention, the formulation can be formulated in the form of water-soluble powder or granule. Such water-soluble powder or granules can be compressed in tablets or are made available as packaged in suitable bottles or packages optionally. No matter in which dosage form the final product is made available, it is dissolved in water before use and administered through oral route.

The object of the invention, the formulations can be manufactured by any of the methods disclosed in the prior art. These methods include dry granulation, wet granulation, dry blending and direct compression.

The method preferred for the manufacturing of the formulations based on the present invention is dry blending method. Due to dry blending method, time and cost advantages were gained and problems that may be experienced with complex manufacturing methods were handled.

The formulation of the object of the invention is preferably manufactured by a method consisting of the following steps:
- Paracetamol is passed through a sieve with a mesh size of 0.4 mm;
- Citric acid anhydride and sodium citrate anhydride are passed through a sieve with a mesh size of 2 mm and taken into a container;
- Paracetamol sieved in the first step is added to the container;
- Mannitol is sieved and its 10% in weight is separated. Phenylephrine hydrochloride, pheniramine maleate, ascorbic acid and sweetener, flavoring and coloring agents are added to the separated part and it is blended for 5 minutes,
- The mixture obtained in the previous step is added to the container. The blending bag is washed with the remaining part of mannitol and it is transferred to the container,
- The container is blended for 40 minutes,
- The obtained mixture acquired is converted into suitable dosage form. For this purpose, the formulation may be either compressed in tablets or filled into suitable bottles or packages. The present invention will be described in more detail by way of the following examples, but scope of the present invention is not limited thereto.

### Example-1:

Paracetamol with a D90 value of 223 µm, citric acid anhydride and sodium citrate anhydride are passed through a sieve with a mesh size of 2 mm and taken into a container. Then mannitol, phenylephrine hydrochloride, pheniramine maleate, ascorbic acid, sucralose, lemon aroma are added to the mixture and the container is blended for 45 minutes. The mixture is packaged as suitable package.

### Example-2 (reference):

Paracetamol with a D90 value of 465 µm, citric acid anhydride and sodium citrate anhydride are passed through a sieve with a mesh size of 2 mm and taken into a container. Then mannitol, phenylephrine hydrochloride, pheniramine maleate, ascorbic acid, sucralose, lemon aroma are added to the mixture and the container is blended for 45 minutes. The mixture is packaged as suitable package.

In Table-1, the 5^{th} min. and 10^{th} min. content uniformity results and initial assay results of the samples, which were blended at 400 rpm after adding 200 mL of water at 50 °C to the Example-1, Example-2 and NeoCitran Grippe® sachet contents, are presented comparatively. Also, while the Example-1 solution has a clear appearance, the Example-2 and NeoCitran Grippe® solutions have opaque appearances.

In accordance with the formulation object of the invention, paracetamol with a D90 value of 200 to 400 µm was used in Example-1, and paracetamol with a D90 value of more than 400 µm was used in Example-2.

In Table-2, the Initial, 1^{st} month and 3^{rd} month stability results of the Example-1 and NeoCitran Grippe® sachets are presented. In the 3^{rd} month stability study, 75% Relative moisture and 3 different temperatures were used.

The descriptions of the impurity abbreviations presented in Table-2 are as follows.

| | |
|---|---|
| Paracetamol Imp K | 4-aminophenol |
| Paracetamol Imp F | 4-nitrophenol |
| Paracetamol Imp J | N-(4-chlorophenyl)acetamide (chloroacetanilide) |
| Pheniramine Imp A | 2-benzylpyridine |
| Pheniramine Imp B | 4-benzylpyridine |
| Phenylephrine Imp C | 1-(3-hydroxyphenyl)-2-(methyl amino) ethanone |
| Phenylephrine Imp E | 2-(benzylmethylamino)- 1-(3-hydroxyphenyl) ethanone |
| Phenylephrine Imp F | 6-Methoxy-α-methyl-2-naphthalene acetic acid |
| Phenylephrine Imp G | 3-Hydroxy acetophenone |

| **TABLE-1** | | | | | |
|---|---|---|---|---|---|
| | | **Phenylephrine** | **Pheniramine** | **Vitamin C** | **Paracetamol** |
| **Example-1** | Assay - initial (Solvent = Buffer: ACN) | 100,5 | 99,8 | 101,2 | 100 |
| | 5 min - Content Uniformity (Solvent = Water) | 100,1 | 99,4 | 100,5 | **93,3** |
| | 10 min - Content Uniformity (Solvent = Water) | 100,1 | 99,0 | 101,0 | 99,8 |
| **Example -2** | Assay- initial (Solvent = Buffer: ACN) | 94,0 | 97,0 | 96,5 | 98,1 |
| | 5 min - Content Uniformity (Solvent = Water) | 85,0 | 86,0 | 86,2 | **61,3** |
| | 10 min - Content Uniformity (Solvent = Water) | 86,0 | 89,7 | 92,9 | 87,5 |
| **NeoCitran Grippe®** | Assay- initial (Solvent = Buffer: ACN) | 92,6 | 93,0 | 97,1 | 98,6 |
| | 5 min - Content Uniformity (Solvent = Water) | 82,2 | 84,7 | 86,3 | 57,4 |
| | 10 min - Content Uniformity (Solvent = Water) | 86,5 | 90,9 | 92,9 | 89,4 |
| ACN: Acetonitrile | | | | | |

| **TABLE-2** | | | | | | | | | | | |
|---|---|---|---|---|---|---|---|---|---|---|---|
| | | **NeoCitran Grippe®** | | | | | **Example-1** | | | | |
| **Tests** | **Limit** | **Initial** | **1^{st} month 40 °C 75% RH** | **3^{rd} month 40 °C 75% RH** | **3^{rd} month 30 °C 75% RH** | **3^{rd} month 25 °C 75% RH** | **Initial** | **1^{st} month 40 °C 75% RH** | **3^{rd} month 40 °C 75% RH** | **3^{rd} month 30 °C 75% RH** | **3^{rd} month 25 °C 75% RH** |
| **Assay** | | | | | | | | | | | |
| Paracetamol | 95.0% - 105.0% | 98,6 | 96,8 | 100,4 | 98,0 | 98,3 | 100 | 102,3 | 102,0 | 100,5 | 100,6 |
| Phenylephrine | 90.0% - 110.0% | 92,6 | 90,0 | 87,0 | 92,0 | 94,4 | 100,5 | 102,7 | 98,0 | 102,8 | 103,8 |
| Pheniramine | 90.0% - 110.0% | 93,0 | 91,8 | 96,8 | 96,5 | 95,4 | 99,8 | 98,9 | 98,3 | 98,4 | 95,4 |
| Ascorbic acid | 90.0% - 110.0% | 97,1 | 88,1 | 85,0 | 86,3 | 90,9 | 101,2 | 104,6 | 102,7 | 103,3 | 103,3 |

| **Impurities** | | | | | | | | | | | |
|---|---|---|---|---|---|---|---|---|---|---|---|
| Paracetamol Imp K | Max. 0.2% | TE | TE | TE | TE | TE | TE | TE | TE | TE | TE |
| Paracetamol Imp F | Max. 0.2% | 0,001 | 0,002 | TE | TE | TE | TE | TE | TE | TE | TE |
| Paracetamol Imp J | Max. 0.2% | TE | TE | TE | TE | TE | TE | TE | TE | TE | TE |
| Pheniramine Imp A | Max. 0.3% | 0,001 | TE | 0,047 | 0,019 | 0,024 | TE | TE | 0,004 | TE | 0,001 |
| Pheniramine Imp B | Max. 0.3% | 0,005 | TE | TE | TE | TE | TE | TE | TE | TE | TE |
| Phenylephrine Imp C | Max. 0.5% | TE | TE | TE | TE | TE | TE | TE | TE | TE | TE |
| Phenylephrine Imp E | Max. 0.5% | TE | TE | TE | TE | TE | TE | TE | TE | TE | TE |
| Phenylephrine Imp F | Max. 0.5% | TE | TE | TE | TE | TE | TE | TE | TE | TE | TE |
| Phenylephrine Imp G | Max. 0.5% | TE | TE | TE | TE | TE | TE | TE | 0,097 | TE | 0,011 |
| Maximum Unknown Impurity | Max. 0.2% | 0,507 (RRT 1,54) | 0,402 (RRT 1,54) | 2,602 (RRT 1,60) | 3,043 (RRT 1,60) | 2,759 (RRT 1,60) | 0,108 (RRT 3,91) | 0,100 (RRT 1,56) | 0,095 (RRT 3,54) | 0,091 (RRT 3,54) | 0,112 (RRT 1,17) |
| Total Impurity | | 1,464 | 1,676 | 5,486 | 5,689 | 5,079 | 0,278 | 0,362 | 0,684 | 0,384 | 0,540 |
| TE: Not detected. | | | | | | | | | | | |

## Claims

1. A water-soluble stable formulation comprising phenylephrine or a pharmaceutically acceptable salt thereof, pheniramine or a pharmaceutically acceptable salt thereof, paracetamol and ascorbic acid and at least one pharmaceutically acceptable excipient wherein at least one pharmaceutical excipient is mannitol and paracetamol in the formulation has a volume diameter of D₉₀ value of 200 to 400 µm.

2. The water-soluble stable formulation according to Claim 1, wherein said formulation comprises 10 to 1000 mg paracetamol.

3. The water-soluble stable formulation according to Claim 1, wherein said formulation comprises 5 to 50 mg phenylephrine or a pharmaceutically acceptable salt thereof.

4. The water-soluble formulation according to Claim 3, wherein the phenylephrine salt is phenylephrine hydrochloride.

5. The water-soluble stable formulation according to Claim 1, wherein said formulation comprises 10 to 100 mg pheniramine or a pharmaceutically acceptable salt thereof.

6. The water-soluble formulation according to Claim 5, wherein the pheniramine salt is pheniramine maleate.

7. The water-soluble stable formulation according to Claim 1, wherein said formulation comprises 10 to 200 mg ascorbic acid.

8. The water-soluble stable formulation according to claim 1, wherein said formulation comprises:
- paracetamol having a volume diameter of D₉₀ value of 200 to 400 µm,
- phenylephrine hydrochloride,
- pheniramine maleate,
- ascorbic acid,
- citric acid anhydride,
- sodium citrate anhydride,
- mannitol and
- at least one pharmaceutically acceptable excipient.

9. A manufacturing method of the formulation according to Claim 1, wherein said manufacturing method consists of the following steps:
- paracetamol is passed through a sieve with a mesh size of 0.4 mm,
- citric acid anhydride and sodium citrate anhydride are passed through a sieve with a mesh size of 2 mm and taken into a container,
- paracetamol sieved in the first step is added to the container,
- mannitol is sieved and its 10% in weight is separated. Phenylephrine hydrochloride, pheniramine maleate, ascorbic acid, sweetener, flavoring and coloring agents are added to the separated part and it is blended for 5 minutes,
- the obtained mixture in the previous step is added to the container. The blending bag is washed with the remaining part of mannitol and it is transferred to the container,
- the container is blended for 40 minutes and
- the obtained mixture is converted into the suitable dosage form; for this purpose, the formulation may be either compressed in tablets or filled into suitable bottles or packages.

## Patentansprüche

1. Wasserlösliche stabile Zusammensetzung, umfassend Phenylephrin oder ein pharmazeutisch verträgliches Salz davon, Pheniramin oder ein pharmazeutisch verträgliches Salz davon, Paracetamol und Ascorbinsäure und mindestens einen pharmazeutisch verträglichen Hilfsstoff, wobei mindestens ein pharmazeutischer Hilfsstoff Mannitol ist und Paracetamol in der Zusammensetzung einen Volumendurchmesser des D90-Wertes von 200 bis 400 µm aufweist.

2. Die wasserlösliche stabile Zusammensetzung nach Anspruch 1, wobei die Zusammensetzung 10 bis 1000 mg Paracetamol enthält.

3. Die wasserlösliche Zusammensetzung nach Anspruch 1, wobei die Zusammensetzung 5 bis 50 mg Phenylephrin oder ein pharmazeutisch verträgliches Salz davon enthält.

4. Die wasserlösliche Zusammensetzung nach Anspruch 3, wobei das Phenylephrinsalz Phenylephrinhydrochlorid ist.

5. Die wasserlösliche Zusammensetzung nach Anspruch 1, wobei die Zusammensetzung 10 bis 100 mg Pheniramin oder ein pharmazeutisch verträgliches Salz davon enthält.

6. Die wasserlösliche Zusammensetzung nach Anspruch 5, wobei das Pheniraminsalz Pheniraminmaleat ist.

7. Die wasserlösliche Zusammensetzung nach Anspruch 1, wobei die Zusammensetzung 10 bis 200 mg Ascorbinsäure enthält.

8. Die wasserlösliche Zusammensetzung nach Anspruch 1, wobei diese Zusammensetzung folgendes enthält:
- Paracetamol mit einem Volumendurchmesser des D90-Wertes von 200 bis 400 µm,
- Phenylephrinhydrochlorid,
- Pheniraminmaleat,
- Ascorbinsäure,
- Ascorbinsäureanhydrid,
- Natriumcitrat-Anhydrid
- Mannitol und
- mindestens einen pharmazeutisch verträglichen Hilfsstoff.

9. Herstellungsverfahren für die Zusammensetzung nach Anspruch 1, wobei das Herstellungsverfahren die folgenden Schritten umfasst:
- Paracetamol wird durch ein Sieb mit einer Maschenweite von 0,4 mm gesiebt,
- Zitronensäureanhydrid und Natriumcitratanhydrid werden durch ein Sieb mit einer Maschenweite von 2 mm gesiebt und in einen Behälter gefüllt,
- Das im ersten Schritt gesiebte Paracetamol wird in den Behälter gegeben,
- Mannitol wird gesiebt und 10 Gew.-% davon werden abgetrennt. Es werden dem abgetrennten Teil Phenylephrinhydrochlorid, Pheniraminmaleat, Ascorbinsäure, Süßstoff, Aroma- und Farbstoffe zugegeben und 5 Minuten lang gemischt,
- die im vorhergehenden Schritt erhaltene Mischung wird in den Behälter gegeben. Der Mischungsbeutel wird mit dem restlichen Teil des Mannitols gewaschen und in den Behälter überführt,
- der Behälter wird 40 Minuten lang gemischt und
- die erhaltene Mischung wird in die geeignete Darreichungsform überführt; zu diesem Zweck kann die Zusammensetzung entweder in Tabletten komprimiert oder in geeignete Flaschen oder Verpackungen abgefüllt werden.

## Revendications

1. Une formulation stable soluble dans l'eau, comprenant phényléphrine ou un sel pharmaceutiquement acceptable de celle-ci, phéniramine ou un sel pharmaceutiquement acceptable de celle-ci, paracétamol et acide ascorbique et au moins une excipient pharmaceutiquement acceptable, où au moins un excipient pharmaceutique est mannitol et paracétamol a un diamètre en volume D₉₀ de 200 à 400 µm.

2. La formulation stable soluble dans l'eau selon la revendication 1, où ladite formulation comprend 10 à 1000 mg de paracétamol.

3. La formulation stable soluble dans l'eau selon la revendication 1, où ladite formulation comprend 5 à 50 mg de phényléphrine ou un sel pharmaceutiquement acceptable de celle-ci.

4. La formulation soluble dans l'eau selon la revendication 3, où le sel de la phényléphrine est le chlorhydrate de phényléphrine.

5. La formulation stable soluble dans l'eau selon la revendication 1, où ladite formulation comprend 10 à 100 mg de phéniramine ou un sel pharmaceutiquement acceptable de celle-ci.

6. La formulation stable soluble dans l'eau selon la revendication 5, où le sel phéniramine est le maléate de phéniramine.

7. La formulation stable soluble dans l'eau selon la revendication 1, où ladite formulation comprend 10 à 200 mg de l'acide ascorbique.

8. La formulation stable soluble dans l'eau selon la revendication 1, où ladite formulation comprend :
- paracétamol ayant un diamètre en volume D₉₀ de 200 à 400 µm,
- clorhydrate de phényléphrine,
- maléate de phéniramine,
- acide ascorbique,
- anhydride d'acide citrique,
- mannitol et
- au moins un excipient pharmaceutiquement acceptable.

9. Un méthode de fabrication de la formulation selon la revendication 1, où ladite méthode de fabrication se compose des étapes suivantes :
- le paracétamol est passé à travers un tamis d'un maillage de 0,4 mm ;
- l'anhydride d'acide citrique et l'anhydride de citrate de sodium sont passés à travers un tamis avec un tamis d'un maillage de 2 mm et placés dans un récipient ;
- le paracétamol tamisé dans la première étape est ajouté au récipient ;
- le mannitol est tamisé et son 10% en poids sont séparés. Du chlorhydrate de phényléprine, du maléate de phéniramine, de l'acide ascorbique, un édulcorant, des agents aromatisants et colorants sont ajoutés à la partie séparée et elle est mélangée pendant 5 minutes ;
- le mélange obtenu à l'étape précédente est ajouté au récipient. Le sac de mélange est lavé avec la partie restante de mannitol et le contenu est transféré dans le récipient ;
- le récipient est mélangé pendant 40 minutes et
- le mélange obtenu est converti en la forme posologique appropriée; à cet effet, la formulation peut être soit comprimée en comprimés, soit remplie dans des flacons ou emballages appropriés.
